# EUROPEAN PATENT SPECIFICATION

(11) **EP 3 878 359 B1**
(45) Date of publication and mention of the grant of the patent: **11.09.2024**
(21) Application number: 19881385.9
(22) Date of filing: 03.10.2019
(51) Int. Cl.: A61B 5/268, A61B 5/16, A61B 5/291, A61B 5/251, A61B 5/263

(54) **BIOLOGICAL ELECTRODE, BIOLOGICAL SENSOR, AND BIOLOGICAL SIGNAL MEASUREMENT SYSTEM**
BIOLOGISCHE ELEKTRODE, BIOLOGISCHER SENSOR UND BIOLOGISCHES SIGNALMESSSYSTEM
ÉLECTRODE BIOLOGIQUE, CAPTEUR BIOLOGIQUE ET SYSTÈME DE MESURE DE SIGNAL BIOLOGIQUE

(30) Priority: 09.11.2018 JP 2018211725
(43) Date of publication of application: 15.09.2021
(73) Proprietor: Sumitomo Bakelite Co.Ltd., Shinagawa-ku Tokyo 140-0002 (JP)
(72) Inventor: KITAZOE Katsuma, Tokyo 140-0002 (JP); YAGISAWA Takashi, Tokyo 140-0002 (JP); SAITO Takeshi, Tokyo 140-0002 (JP); UENO Masaomi, Tokyo 140-0002 (JP); HARADA Takuya, Tokyo 140-0002 (JP)
(74) Representative: Ter Meer Steinmeister & Partner
(86) International application number: PCT/JP2019/039176
(87) International publication number: WO 2020/095589

(56) References cited:
- WO-A1-2013/073673
- JP-A- 2016 020 463
- JP-A- 2017 074 370
- US-A- 4 967 038
- US-A1- 2016 143 554
- US-A1- 2016 174 859
- US-A1- 2018 235 499

## Description

### TECHNICAL FIELD

The present invention relates to a biomedical electrode, biomedical sensor, and a biomedical signal measurement system.

### BACKGROUND ART

Various biomedical electrodes have been developed until now. As such a technique, for example, a technique described in Patent Document 1 is known. Patent Document 1 describes an electroencephalographic electrode in which a contact portion that is provided at a distal end of a protrusion portion and comes into contact with a scalp during measurement of brain waves is formed of metal (claim 1 and Fig. 2 in Patent Document 1). Patent Document 2 describes an apparatus for measuring bioelectrical signals (an electrode is described in figure 5 and paragraphs [0186] and [0187] in Patent Document 2).

### RELATED DOCUMENT

### PATENT DOCUMENT

[Patent Document 1] Japanese Laid-open patent publication NO. 2013-111361
[Patent Document 2] US 2016/0143554 A1

### SUMMARY OF THE INVENTION

However, as a result of an investigation, the present inventors found that there is a room for improvement of the biomedical electrode described in Patent Document 1 from the viewpoint of wearing stability and measurement stability.

The present inventors conducted an investigation on a biomedical electrode in which a distal end of a protrusion portion (pillar portion) is configured with a metallic portion formed of metal. As a result, when the distal end comes into contact with a measurement target, a subject may feel pain or discomfort, and the metallic portion is not substantially deformed during the contact. Therefore, it was found that there is a concern that the contact area of the distal end does not increase such that the contact resistance cannot be reduced.

On the other hand, the present inventors conducted an investigation on a biomedical electrode in which a distal end of a pillar portion is coated with a conductive resin layer having higher flexibility than a metallic portion. As a result, the distal end coated with the conductive resin layer can alleviate pain or discomfort to a subject and the followability to a measurement target is improved. Therefore, it was found that the contact area with the distal end increases such that the contact resistance can be reduced.

However, in consideration of a configuration in which a region from a distal end to a base end of a pillar portion of a biomedical electrode is coated with a conductive resin layer, it was found that noise may be generated due to disconnection of the conductive resin layer during deformation of the pillar portion or a variation in internal resistance of the conductive resin layer caused by elongation and contraction of the pillar portion.

The present inventors further conducted a thorough investigation based on the findings and found that disconnection or noise generation caused by deformation can be suppressed by electrically connecting a distal end of a pillar portion and a conductive resin layer in a state where the distal end is coated with the conductive resin layer and arranging a conductive wire in the pillar portion from a distal end side toward a base end side, thereby completing the present invention.

According to the present invention, there is provided a biomedical electrode in accordance with claim 1.

In addition, according to the present invention, there is provided a biomedical sensor including the above-described biomedical electrode.

In addition, according to the present invention, there is provided a biomedical signal measurement system including the above-described biomedical sensor.

### ADVANTAGEOUS EFFECTS OF INVENTION

The present invention provides a biomedical electrode having excellent wearing stability and measurement stability, and a biomedical sensor and a biomedical signal measurement system including the biomedical electrode.

### BRIEF DESCRIPTION OF THE DRAWINGS

The above-described object and other objects, characteristics, and advantageous effects will be further clarified using the following preferred embodiment and the following accompanying drawings.

Figs. 1A and 1B are schematic diagrams showing the summary of an example of a biomedical electrode according to an embodiment, in which Fig. 1A is a perspective view and Fig. 1B is a cross-sectional view taken along line A-A of Fig. 1A.
Figs. 2A and 2B are schematic diagrams showing the summary of an example of the biomedical electrode according to the embodiment, in which Fig. 2A is a perspective view and Fig. 2B is a cross-sectional view taken along line A-A of Fig. 2A.
Figs. 3A to 3F are cross-sectional views schematically showing a modification example of a conductive wire according to the embodiment.
Fig. 4 is a schematic diagram showing the summary of a biomedical sensor according to an embodiment.

### DESCRIPTION OF EMBODIMENTS

An embodiment will be described by defining front, rear, left, right, upper, and lower directions as shown in the drawings. However, the directions are defined for convenience of easy understanding of a relationship between components. Accordingly, this definition does not limit directions during the manufacturing or use of a product according to the present invention.

In all the drawings, the same components will be represented by the same reference numerals, and the description thereof will not be repeated. In addition, the drawings are schematic diagrams, in which a dimensional ratio does not match the actual one.

In this specification, the term "substantially" includes a range in consideration of a tolerance, a variation, or the like during manufacturing unless explicitly specified otherwise. A range represented by "to" includes an upper limit value and a lower limit value unless explicitly specified otherwise.

A biomedical electrode according to an embodiment will be briefly described.

The biomedical electrode includes: a plate-shaped support portion; an elastic pillar portion that is provided on a first surface of the plate-shaped support portion; a conductive resin layer that is formed to cover a distal end of the elastic pillar portion; and a conductive wire that is electrically connected to the conductive resin layer and is arranged in the elastic pillar portion from a distal end side toward a base end side.

The present inventors obtain the following findings.

Until now, a configuration in which electrical connection of the pillar portion of the biomedical electrode is realized with only the conductive resin layer has been considered. In this case, adoption of a configuration the surface of the pillar portion from the distal end to the base end is covered with the conductive resin layer can be considered.

However, it was found that, for this configuration, there is a room for improvement in measurement stability.

The detailed mechanism is not clear but is presumed to be that, when the pillar portion comes into contact with a measurement target, noise may be generated due to disconnection of the conductive resin layer during deformation of the pillar portion or a variation in internal resistance of the conductive resin layer caused by elongation and contraction of the pillar portion.

On the other hand, a configuration in which the electrical connection of the pillar portion of the biomedical electrode is realized with both the conductive resin layer and the conductive wire instead of only the conductive resin layer formed on the surface was investigated. This conductive wire is arranged in the pillar portion from the distal end side toward the base end side such that it can be electrically connected to the conductive resin layer on the distal end side.

When a plurality of pillar portions of the biomedical electrode come into contact with a measurement target, the pillar portions are elastically deformed so as to spread toward the outside. That is, the outside of the pillar portion is deformed in a contraction direction, and the inside thereof is deformed in a tension direction. At this time, the internal deformation or deformation strain is less than that of the deformation on the surface of the pillar portion.

Accordingly, as a result of investigation, it was found that the conductive wire arranged in the pillar portion is less likely to break as compared to the conductive resin layer formed on the surface of the pillar portion, and a variation in internal resistance caused by deformation is also reduced.

Accordingly, in the biomedical electrode according to the embodiment including both the conductive resin layer and the conductive wire, disconnection or noise generation caused by deformation of the pillar portion can be suppressed as compared to the configuration where only the conductive resin layer is adopted, and thus the measurement stability can be improved.

As a result of investigation by the present inventors, when metal, graphite, a plating film or a ceramic coated with carbon is used for the distal end of the pillar portion of the biomedical electrode, the distal end portion is configured to be hard. Therefore, it is expected that, as in a case where an iron ball is provided for the distal end, the subject may feel pain or discomfort.

In addition, the conductive resin layer formed at the distal end of the pillar portion of the biomedical electrode has higher flexibility than a metallic portion formed of metal, and the followability to a measurement target is also configured to be high. Therefore, the biomedical electrode according to the embodiment can realize the structure having excellent wearing stability and excellent measurement stability.

The biomedical electrode according to the embodiment can detect a variation in bioelectrical potential such as brain wave, heart beat, muscular activity, or nervous system activity. The biomedical electrode can further include a connector or an electronic component to configure a biomedical sensor that can be connected to an external apparatus. This biomedical sensor is wearable. By analyzing the bioelectrical potential such as a brain wave detected from the biomedical sensor, a biomedical signal measurement system corresponding to various uses can be constructed.

Hereinafter, the biomedical electrode according to the embodiment will be described below.

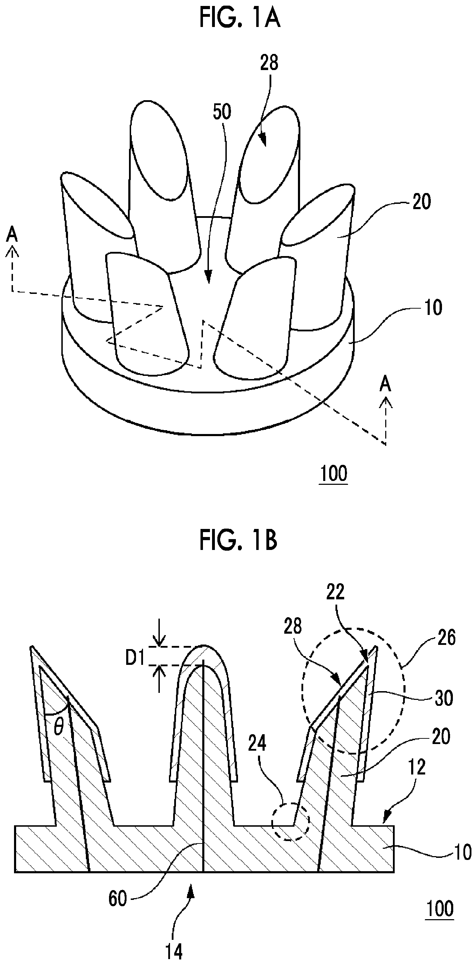

Figs. 1A and 1B are schematic diagrams showing the summary of a biomedical electrode 100 according to the embodiment, in which Fig. 1A is a perspective view and Fig. 1B is a cross-sectional view taken along line A-A of Fig. 1A.

As shown in Figs. 1A and 1B, an example of the biomedical electrode 100 includes a plate-shaped support portion 10, pillar portions (elastic pillar portions) 20, and a conductive resin layer 30.

The plate-shaped support portion 10 is formed of an insulating elastic member, in which one pillar portion 20 or two or more pillar portions 20 may be provided on a first surface 12. The pillar portion 20 is formed of an insulating elastic member. The conductive resin layer 30 is formed of a conductive elastic member and may be formed to cover at least a surface of a distal end 22 (a part of a distal end portion 26) of the pillar portion 20. A conductive wire 60 is arranged in the pillar portion 20 to be electrically connected to the conductive resin layer 30.

When the distal end portion 26 of the biomedical electrode 100 comes into contact with a measurement target, a bioelectric signal detected by the pillar portions 20 through the conductive resin layer 30 and the conductive wire 60 can be transmitted to a connector (external connection portion 110) provided in the plate-shaped support portion 10. The bioelectric signal detected by the biomedical electrode 100 can be transmitted to an external apparatus through the connector.

As the conductive wire 60, a well-known material can be used. For example, the conductive wire 60 can be formed of conductive fiber.

As the conductive fiber, one or more selected from the group consisting of metal fiber, metal-coated fiber, carbon fiber, conductive polymer fiber, conductive polymer-coated fiber, and conductive paste-coated fiber can be used. Among these, one kind may be used alone, or two or more kinds may be used in combination.

The metal material of the metal fiber and the metal-coated fiber is not particularly limited as long as it has conductivity. Examples of the metal material include copper, silver, gold, nickel, tin, lead, zinc, bismuth, antimony, stainless steel, aluminum, and an alloy thereof. Among these, one kind may be used alone, or two or more kinds may be used in combination. Among these, from the viewpoint of conductivity, silver can be used. In addition, it is preferable that the metal material does not include metal such as chromium that imposes burden on the environment.

The fiber material of the metal-coated fiber, the conductive polymer-coated fiber, or the conductive paste-coated fiber is not particularly limited and may be any one of synthetic fiber, semisynthetic fiber, or natural fiber. Among these, for example, polyester, nylon, polyurethane, silk, or cotton is preferably used. Among these, one kind may be used alone, or two or more kinds may be used in combination.

Examples of the carbon fiber include a PAN-based carbon fiber and a pitch-based carbon fiber.

As the conductive polymer material of the conductive polymer fiber or the conductive polymer-coated fiber, for example, polythiophene, polypyrrole, polyaniline, polyacetylene, polyphenylene vinylene, polynaphthalene, a mixture of the conductive polymer and the binder resin, for example, a derivatives thereof, or an aqueous solution of the conductive polymer PEDOT-PSS ((3,4-ethylenedioxythiophene)-poly(styrene sulfonate)) is used.

The resin material in the conductive paste of the conductive paste-coated fiber is not particularly limited and preferably has elasticity. For example, the resin material includes one or more selected from the group consisting of silicone rubber, urethane rubber, fluorine rubber, nitrile rubber, acrylic rubber, styrene rubber, chloroprene rubber, and ethylene propylene rubber. Among these, one kind may be used alone, or two or more kinds may be used in combination.

The conductive filler in the conductive paste of the conductive paste-coated fiber is not particularly limited, and a well-known conductive material may be used. For example, the conductive filler may include one or more selected from the group consisting of metal particles, metal fiber, metal-coated fiber, carbon black, acetylene black, graphite, carbon fiber, carbon nanotube, a conductive polymer, conductive polymer-coated fiber, and metal nanowire.

The metal forming the conductive filler is not particularly limited. For example, the metal may include at least one or two or more among copper, silver, gold, nickel, tin, lead, zinc, bismuth, antimony, and alloys thereof. In particular, silver or copper is preferable from the viewpoint of high conductivity or high availability.

The conductive wire 60 may be formed of twisted yarn obtained by twisting a plurality of linear conductive fibers. As a result, disconnection of the conductive wire 60 during deformation can be suppressed.

In this specification, the coating of the conductive fiber represents not only that the outer surface of the fiber material is covered with the conductive fiber but also that gaps between fibers in twisted yarn obtained by twisting single fibers are impregnated with metal, the conductive polymer, or the conductive paste such that each of the single fibers forming the twisted yarn is coated with the conductive fiber.

The tensile elongation at break of the conductive wire 60 is, for example, 1% or higher and 50% or lower and preferably 1.5% or higher and 45% or lower. In the numerical range, excessive deformation of the pillar portion 20 can be suppressed while suppressing break during deformation.

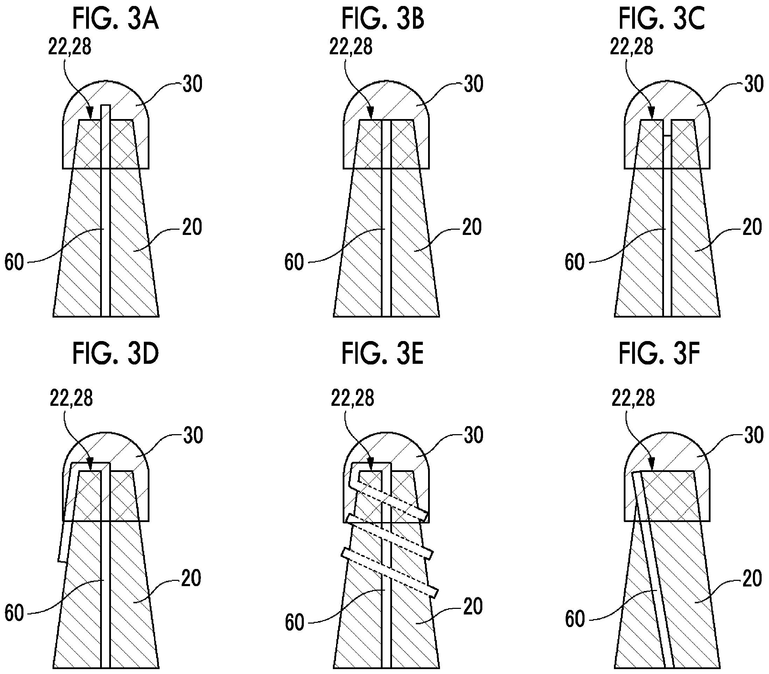

The conductive wire 60 can adopt various arrangement structures as long as the inside of the pillar portion 20 is electrically connected. Examples of the arrangement structure of the conductive wire 60 will be shown in Figs. 3A to 3F.

The distal end of the conductive wire 60 may adopt any one of structures (Figs. 3A, 3D and 3E) that protrude from the distal end 22 or an inclined surface 28 of the pillar portion 20, a structure (Fig. 3B) that substantially flushes with the distal end 22 or the inclined surface 28, and a structure (Fig. 3C) that is buried in the distal end 22 or the inclined surface 28. From the viewpoint of connection stability to the conductive resin layer 30, the structure that protrudes from the distal end 22 or the inclined surface 28 can be adopted.

A part or the entirety of the protrusion portion of the conductive wire 60 to the distal end is covered with the conductive resin layer 30.

As the discharge structure of the conductive wire 60 to the distal end, a non-folded structure (Fig. 3A), a folded structure (Fig. 3D), or a structure (Fig. 3E) wound around the surface of the distal end portion of the pillar portion 20 can be adopted. In addition, the conductive wire 60 may be inclined with respect to a central axis of the pillar portion 20 without passing through the central axis (Fig. 3F).

Another end of the conductive wire 60 opposite to the distal end may have any configuration as long as it can be connected to the connector electrically connected to a second surface 14 of the plate-shaped support portion 10. For example, the other end of the conductive wire 60 may pass through a base end portion 24 of the pillar portion 20 and may extend up to the first surface 12,a side surface, or the second surface 14 side of the plate-shaped support portion 10. The other end of the conductive wire 60 may be electrically connected to the connector through the conductive resin layer 30 provided on the second surface 14.

The conductive resin layer 30 may be configured to cover at least the surface of the distal end 22 of the pillar portion 20, or may be configured to cover a region of the pillar portion 20 from the distal end 22 to the distal end portion 26 or from the distal end 22 to an intermediate portion of the base end portion 24. That is, the conductive resin layer 30 may be configured not to cover the entire surface of the pillar portion 20.

The conductive resin layer 30 may be configured to cover the first surface 12 or the second surface 14 of the plate-shaped support portion 10 in a state where it is spaced from the portion covering the distal end 22.

When an overall length (the distance from the distal end 22 to the base end portion 24) of the elastic pillar portion 20 is represented by L, the conductive resin layer 30 may be formed in a region of, for example, 8/10L or less, preferably 7/10L or less, and more preferably 6/10L or less from the distal end 22 of the elastic pillar portion 20. As a result, the costs can be reduced.

On the other hand, when the overall length of the elastic pillar portion 20 is represented by L, the conductive resin layer 30 may be formed in a region of, for example, 1/10L or more, preferably 2/10L or more, and more preferably 3/10L or more from the distal end 22 of the elastic pillar portion 20. As a result, the connection stability to the conductive wire 60 can be improved. In addition, by forming the conductive resin layer 30 having a certain area in the distal end portion of the pillar portion 20, the contact resistance can be reduced.

The shape of the plate-shaped support portion 10 in a top view may be, for example, a substantially circular shape such as an elliptical shape or a perfect circular shape or may be a substantially polygonal shape such as a square shape, a rectangular shape, a pentagonal shape, or a hexagonal shape. Roundness (R) may be imparted to a corner portion of the polygonal shape.

Here, the top view represents being observed from the top when seen from the distal end 22 of the pillar portion 20 toward the plate-shaped support portion 10.

The first surface 12 of the plate-shaped support portion 10 may be configured to be flat or have a curved surface in a part or the entirety thereof.

Here, the first surface 12 may be configured with a surface that passes through at least three contact points in contact with a side surface of the pillar portion 20 and the plate-shaped support portion 10. In addition, the plate-shaped support portion 10 and the pillar portion 20 may be seamlessly configured without an interface therebetween.

A second surface 14 of the plate-shaped support portion 10 may have a structure that can be connected to the connector. For example, an electrode that can be electrically connected to the connector may be buried in the second surface 14 opposite to the first surface 12 in a state where a part of the second surface 14 is exposed.

In addition, at least a part or the entirety of the second surface 14 may be covered with a conductive elastic member. At this time, a side surface of the plate-shaped support portion 10 may not be covered with a conductive elastic member.

The plate-shaped support portion 10 may be configured as a member integrated with the pillar portion 20. That is, the plate-shaped support portion 10 may be integrally formed of the same resin material as the pillar portion 20. For example, by molding a curable elastomer composition such as a silicone rubber-based curable composition described below, the plate-shaped support portion 10 and the plurality of pillar portions 20 may be seamlessly bonded to each other to obtain a molded body. As a result, an elastic molded body having excellent flexibility and strength can be realized.

Each of the plate-shaped support portion 10 and the pillar portion 20 may be formed of an insulating silicone rubber (rubber molded body) including a silicone rubber without including a conductive filler as an insulating elastic member.

One or more pillar portions 20 may be provided to protrude from the first surface 12 of the plate-shaped support portion 10.

The shape of the pillar portion 20 in a top view may be a substantially circular shape such as an elliptical shape or a perfect circular shape or may be a substantially polygonal shape such as a square shape, a rectangular shape, a pentagonal shape, or a hexagonal shape.

It is preferable that the plurality of pillar portions 20 are arranged to surround a center portion 50 of the first surface 12 of the plate-shaped support portion 10. In other words, the plurality of pillar portions 20 are arranged along an outer peripheral edge of the plate-shaped support portion 10. As a result, the followability of the pillar portion 20 to a living body can be improved.

Here, the center portion 50 may be a region including a center of gravity position of the first surface 12 when seen from a direction perpendicular to the first surface 12. In addition, when the shortest distance from the center of gravity position of the first surface 12 to a circumference of the first surface 12 is represented by Dmin, the center portion 50 is preferably 2/10 Dmin or less from the center of gravity position and may be 1/10 Dmin or less from the center of gravity position.

In a case where the shape of the first surface 12 of the plate-shaped support portion 10 in a top view is a substantially circular shape, the plurality of pillar portions 20 may be arranged on the first surface 12 in a substantially circular shape or a substantially elliptical shape. The pillar portions 20 may be arranged to configure one or more concentric circles around the center portion 50. As a result, when coming into contact with a measurement target, the distal end portion 26 of the pillar portion 20 substantially uniformly spreads in a radial direction. Therefore, the measurement stability can be improved.

The center portion 50 of the plate-shaped support portion 10 can be positioned at substantially equal distances from the distal ends 22 of the plurality of pillar portions 20 present on the same circumference.

The pillar portion 20 may have a structure in which the center is eccentric with respect to the direction perpendicular to the first surface 12 of the plate-shaped support portion 10. From the viewpoint of manufacturing stability, it is preferable that the central axis of the pillar portion 20 having the eccentric structure is inclined from the center portion 50 of the plate-shaped support portion 10 toward the outside.

The inclination of the central axis of the pillar portion 20 refers to an outside angle (acute angle) between the central axis of the pillar portion 20 and the first surface 12 (surface) of the plate-shaped support portion 10 in a cross-sectional view passing through the center portion 50 and the center portion of the pillar portion 20 from the inside toward the outside of each of the pillar portions 20 with respect to the center portion 50.

The inclination of the central axis of the pillar portion 20 is, for example, 45 degrees to 90 degrees, preferably 50 degrees to 88 degrees, and more preferably 60 degrees to 85 degrees. In the above-described numerical range, releasability from a mold can be improved.

As shown in Fig. 1B, the distal end portions 26 of the plurality of pillar portions 20 may have the inclined surface 28 that is configured such that the height of the pillar portion 20 on the outside is higher than that on the inside in a cross-sectional view in a direction passing through the outside from the inside of each of the pillar portions 20, for example, in a radial direction. The inclined surface 28 of each of the pillar portions 20 may be configured to face the center portion 50 of the plate-shaped support portion 10. When the pillar portion 20 and a measurement target come into contact with each other, the inclined surface 28 can follow a measurement surface of the measurement target. Therefore, a variation in contact area can be suppressed.

The pillar portion 20 including the distal end portion 26 may be configured in a substantially truncated conical shape or a substantially truncated pyramid shape. In particular, from the viewpoint of measurement stability, a substantially truncated conical shape is preferable. The truncated conical pillar portion 20 is configured such that the diameter decreases from a connection portion (base end portion 24) side to the plate-shaped support portion 10 toward the distal end 22 side. Therefore, the manufacturing stability during metallic molding can be improved. In addition, an inner side surface of the pillar portion 20 has a tapered shape that spreads from the inside toward the outside when seen in the pressing direction. Therefore, a variation in the deformation of the pillar portion 20 can be further suppressed.

The shape of the inclined surface 28 in a top view may be, for example, a substantially elliptical shape. This substantially elliptical shape may have a major axis of in a radial direction of a substantially circumference where the pillar portion 20 is arranged. The followability to the measurement surface can be improved.

As shown in Fig. 1B, in a cross-sectional view passing through the outside from the inside of each of the pillar portions 20, an inclination angle θ of the inclined surface 28 refers to an angle between an outside surface of the pillar portion 20 and the inclined surface 28.

The inclination angle θ of the inclined surface 28 is, for example, 10 to 89 degrees, preferably 15 degrees to 80 degrees, more preferably 20 degrees to 60 degrees, and still more preferably 25 degrees to 50 degrees. By adjusting the inclination angle θ of the inclined surface 28 to be the lower limit value or higher, the followability to the measurement surface can be improved. By adjusting the inclination angle θ of the inclined surface 28 to be the upper limit value or lower, a variation in the deformed state can be suppressed.

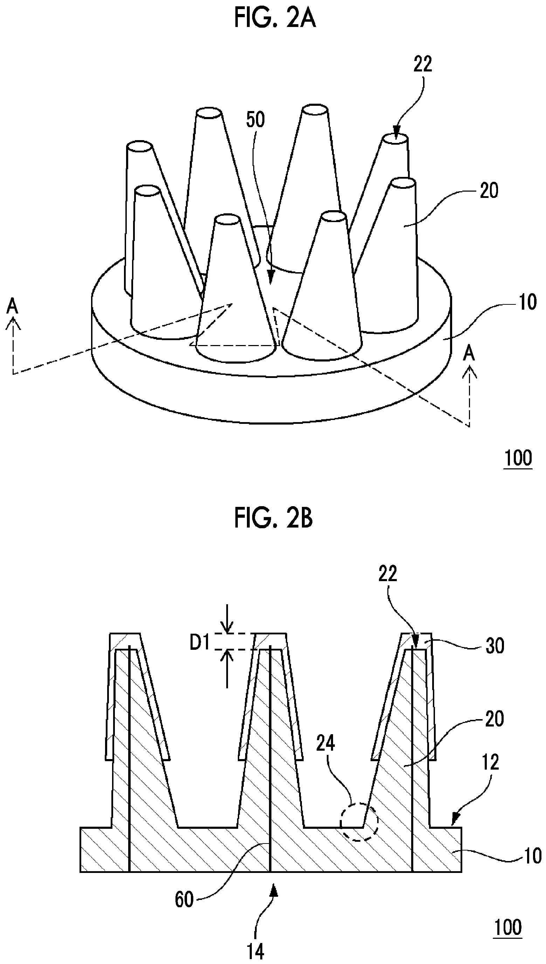

The pillar portion 20 may be configured not to include the above-described inclined surface 28 as shown in Fig. 2B. The pillar portion 20 not including the inclined surface 28 may be formed in a substantially pillar shape, a substantially prismatic shape, a substantially conical shape, a substantially pyramid shape, a substantially truncated conical shape, or a substantially truncated pyramid shape. In particular, a structure having a tapered shape such as a conical shape or a pyramid shape is preferable from the viewpoint of manufacturing stability, and a substantially truncated conical shape is preferable from the viewpoint of measurement stability. The truncated conical pillar portion 20 is configured such that the diameter decreases from a connection portion (base end portion 24) to the plate-shaped support portion 10 toward the distal end 22 side. A shape of the distal end 22 in a top view may be, for example, substantially circular or substantially polygonal.

In a cross-sectional view passing through the center portion 50 and the pillar portion 20, the length of the pillar portion 20 from the base end portion 24 connected to the plate-shaped support portion 10 to the distal end 22 can be configured to be longer than the width of the pillar portion in the base end portion 24. As a result, the arrangement density of the pillar portions 20 can be improved. In addition, the pillar portions 20 can be suppressed from coming into contact with each other during deformation.

The conductive resin layer 30 is formed of a conductive silicone rubber including a conductive filler and a silicone rubber as a conductive elastic member. For example, a conductive solution (conductive silicone rubber-based curable composition) in which a conductive filler is added to an insulating silicone rubber-based curable composition not including a conductive filler described below is applied to the above-described molded body such that the conductive resin layer 30 can be formed. By using the same silicone rubber material as the silicone rubber forming the plate-shaped support portion 10 or the pillar portion 20, the adhesion of the conductive resin layer 30 can be improved.

As the conductive filler, a well-known conductive material may be used. The conductive filler may include one or more selected from the group consisting of metal particles, silver or silver chloride particles, metal fiber, metal-coated fiber, carbon black, acetylene black, graphite, carbon fiber, carbon nanotube, a conductive polymer, conductive polymer-coated fiber, and metal nanowire.

The metal forming the conductive filler is not particularly limited. For example, the metal may include at least one or two or more among copper, silver, gold, nickel, tin, lead, zinc, bismuth, antimony, silver or silver chloride, and alloys thereof. In particular, silver or copper is preferable from the viewpoint of high conductivity or high availability.

The lower limit value of the content of the conductive filler is, for example, 30 mass% or higher, preferably 35 mass% or higher, and more preferably 40 mass% or higher with respect to 100 mass% of the silicone rubber in the conductive resin layer 30. As a result, even in the case of a thin film, the transmission performance of a bioelectric signal can be improved. On the other hand, the upper limit value of the content of the conductive filler is, for example, 90 mass% or lower, preferably 85 mass% or lower, and more preferably 80 mass% or lower with respect to 100 mass% of the silicone rubber in the conductive resin layer 30. As a result, the durability of the conductive resin layer 30 for the deformation of the pillar portion 20 can be improved.

The lower limit value of the thickness of the conductive resin layer 30 is, for example, 5 pm or more, preferably 8 pm or more, and more preferably 10 pm or more. As a result, the durability during repeated use can be improved. On the other hand, the upper limit value of the thickness of the conductive resin layer 30 is, for example, 200 um or less, preferably 150 µm or less, more preferably 100 µm or less, and still more preferably 50 µm or less. As a result, the deformation easiness of the pillar portion 20 can be maintained. In a cross-sectional view of the pillar portion 20, the thickness of the conductive resin layer 30 on at least a part of the distal end 22 or the side surface of the pillar portion 20 is preferably in the numerical range.

Regarding the thickness of the conductive resin layer 30, a thickness D1 on the surface of the distal end 22 of the pillar portion 20 may be configured to be thicker than the thickness of the conductive resin layer 30 on the base end portion 24 side. For example, a part of the distal end 22 side of the pillar portion 20 may be dipped (dip coating) in the paste-like conductive solution. It is preferable that this thick region is provided in the entirety of the distal end portion of the pillar portion 20 in the circumferential direction. As a result, peeling of the conductive resin layer 30 in the distal end portion can be suppressed, and damages such as disconnection of the pillar portion 20 can be suppressed. Therefore, the durability of the biomedical electrode 100 can be improved.

Here, the silicone rubber-based curable composition will be described.

The silicone rubber can be formed of a cured product of the silicone rubber-based curable composition. A step of curing the silicone rubber-based curable resin composition is performed by heating (primary curing) the composition, for example, at 100°C to 250°C for 1 minute to 30 minutes and post-baking (secondary curing) the heated composition at 200°C for 1 hour to 4 hours.

The silicone rubber-based curable composition according to the embodiment may include a vinyl group-containing organopolysiloxane (A). The vinyl group-containing organopolysiloxane (A) is a polymer including the silicone rubber-based curable composition according to the embodiment as a main element.

The vinyl group-containing organopolysiloxane (A) may include a vinyl group-containing linear organopolysiloxane (A1) having a linear structure.

The vinyl group-containing linear organopolysiloxane (A1) has a linear structure and includes a vinyl group, in which the vinyl group functions as a crosslinking point during curing.

The content of the vinyl group in the vinyl group-containing linear organopolysiloxane (A1) is not particularly limited. For example, the vinyl group-containing linear organopolysiloxane (A1) includes two or more vinyl groups in the molecule, and the content thereof is preferably 15 mol% or lower and more preferably 0.01% to 12 mol%. As a result, the content of the vinyl group in the vinyl group-containing linear organopolysiloxane (A1) can be optimized, and a network between the respective components can be reliably formed. In the embodiment, a range represented by "to" includes numerical values of both ends.

In this specification, the content of the vinyl group represents mol% of a vinyl group-containing siloxane unit with respect to 100 mol% of all the units forming the vinyl group-containing linear organopolysiloxane (A1). In this case, it is assumed that one vinyl group is present for each vinyl group-containing siloxane unit.

In addition, the polymerization degree of the vinyl group-containing linear organopolysiloxane (A1) is not particularly limited and is, for example, preferably in a range of about 1000 to 10000 and more preferably in a range of about 2000 to 5000. The polymerization degree can be obtained as, for example, a number average polymerization degree (number average molecular weight) in terms of polystyrene in GPC (gel permeation chromatography) in which chloroform is used as an eluent.

Further, the specific gravity of the vinyl group-containing linear organopolysiloxane (A1) is not particularly limited and is preferably in a range of about 0.9 to 1.1.

By using the vinyl group-containing linear organopolysiloxane (A1) having the polymerization degree and the specific gravity in the above-described ranges, the heat resistance, flame retardancy, chemical stability, and the like of the obtained silicone rubber can be improved.

It is preferable that the vinyl group-containing linear organopolysiloxane (A1) has a structure represented by the following Formula (1).

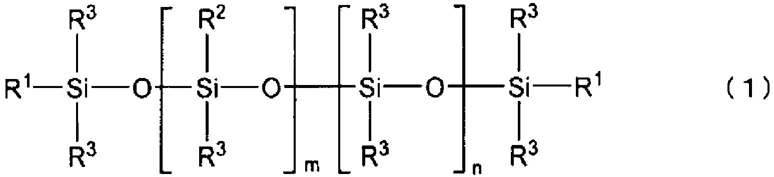

In Formula (1), R¹ represents a substituted or unsubstituted alkyl group, alkenyl group, or aryl group having 1 to 10 carbon atoms, or a hydrocarbon group including a combination thereof. Examples of the alkyl group having 1 to 10 carbon atoms include a methyl group, an ethyl group, and a propyl group. In particular, a methyl group is preferable. Examples of the alkenyl group having 1 to 10 carbon atoms include a vinyl group, an allyl group, and a butenyl group. In particular, a vinyl group is preferable. Examples of the aryl group having 1 to 10 carbon atoms include a phenyl group.

In addition, R² represents a substituted or unsubstituted alkyl group, alkenyl group, or aryl group having 1 to 10 carbon atoms, or a hydrocarbon group including a combination thereof. Examples of the alkyl group having 1 to 10 carbon atoms include a methyl group, an ethyl group, and a propyl group. In particular, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

In addition, R³ represents a substituted or unsubstituted alkyl group or aryl group having 1 to 8 carbon atoms, or a hydrocarbon group including a combination thereof. Examples of the alkyl group having 1 to 8 carbon atoms include a methyl group, an ethyl group, and a propyl group. In particular, a methyl group is preferable. Examples of the aryl group having 1 to 8 carbon atoms include a phenyl group.

Further, examples of a substituent of R¹ and R² in Formula (1) include a methyl group and a vinyl group. Examples of a substituent of R³ include a methyl group.

In Formula (1), a plurality of R¹' s may be independent from each other and may be the same as or different from each other. Further, the same can be applied to R² and R³.

Further, m and n each independently represent the number of repeating units forming the vinyl group-containing linear organopolysiloxane (A1) represented by Formula (1), m represents an integer of 0 to 2000, and n represents an integer of 1000 to 10000. m represents preferably 0 to 1000, and n represents preferably 2000 to 5000.

In addition, examples of a specific structure of the vinyl group-containing linear organopolysiloxane (A1) represented by Formula (1) include a structure represented by the following Formula (1-1).

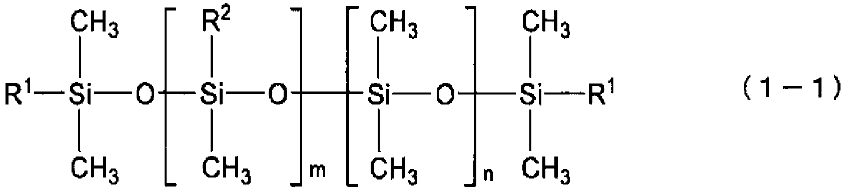

In Formula (1-1), R¹ and R² each independently represent a methyl group or a vinyl group, and at least one of R¹ or R² represents a vinyl group.

Further, it is preferable that as the vinyl group-containing linear organopolysiloxane (A1), a first vinyl group-containing linear organopolysiloxane (A1-1) having two or more vinyl groups in the molecule in which the vinyl group content is 0.4 mol% or lower; or a second vinyl group-containing linear organopolysiloxane (A1-2) in which the vinyl group content is 0.5 to 15 mol% is included. By using the first vinyl group-containing linear organopolysiloxane (A1-1) having the general vinyl group content and the second vinyl group-containing linear organopolysiloxane (A1-2) having the higher vinyl group content in combination as raw rubber that is a material of the silicone rubber, the vinyl groups can be distributed, and a structure of the crosslinking density in the crosslinked network of the silicone rubber can be more effectively formed. As a result, the tear strength of the silicone rubber can be more effectively improved.

Specifically, as the vinyl group-containing linear organopolysiloxane (A1), for example, the first vinyl group-containing linear organopolysiloxane (A1-1) having two or more of a unit in which R¹ in Formula (1-1) represents a vinyl group and/or a unit in which R² in Formula (1-1) represents a vinyl group in the molecule and the content is 0.4 mol% or lower, or the second vinyl group-containing linear organopolysiloxane (A1-2) including 0.5 to 15 mol% of a unit in which R¹ in Formula (1-1) represents a vinyl group and/or a unit in which R² in Formula (1-1) represents a vinyl group is preferably used.

In addition, in the first vinyl group-containing linear organopolysiloxane (A1-1), the vinyl group content is preferably 0.01 to 0.2 mol%. In addition, in the second vinyl group-containing linear organopolysiloxane (A1-2), the vinyl group content is preferably 0.8 to 12 mol%.

Further, in a case where the first vinyl group-containing linear organopolysiloxane (A1-1) and the second vinyl group-containing linear organopolysiloxane (A1-2) are mixed in combination, the ratio between (A1-1) and (A1-2) is not particularly limited. For example, the weight ratio (A1-1): (A1-2) is preferably 50:50 to 95:5 and more preferably 80:20 to 90:10.

As each of the first and second vinyl group-containing linear organopolysiloxanes (A1-1) and (A1-2), only one kind may be used, or two or more kinds may be used in combination.

In addition, the vinyl group-containing organopolysiloxane (A) may include a vinyl group-containing branched organopolysiloxane (A2) having a branched structure.

### <<Organohydrogen Polysiloxane (B)>>

The silicone rubber-based curable composition according to the embodiment may include an organohydrogen polysiloxane (B).

The organohydrogen polysiloxane (B) is classified into a linear organohydrogen polysiloxane (B1) having a linear structure and a branched organohydrogen polysiloxane (B2) having a branched structure and may include either or both of (B1) and (B2).

The linear organohydrogen polysiloxane (B1) is a polymer that has a linear structure and a structure (=Si-H) in which hydrogen is directly bonded to Si and that is obtained by a hydrosilylation reaction of the vinyl group in the vinyl group-containing organopolysiloxane (A) and a vinyl group in a component mixed in the silicone rubber-based curable composition to crosslink the components.

The molecular weight of the linear organohydrogen polysiloxane (B1) is not particularly limited. For example, the weight average molecular weight is preferably 20000 or lower and more preferably 1000 or higher and 10000 or lower.

The weight average molecular weight of the linear organohydrogen polysiloxane (B1) can be measured in terms of polystyrene in GPC (gel permeation chromatography) in which chloroform is used as an eluent.

In addition, it is preferable that, typically, the linear organohydrogen polysiloxane (B1) does not have a vinyl group. As a result, the crosslinking reaction in the molecule of the linear organohydrogen polysiloxane (B1) can be reliably prevented from progressing.

It is preferable to use the linear organohydrogen polysiloxane (B1), for example, having a structure represented by the following Formula (2).

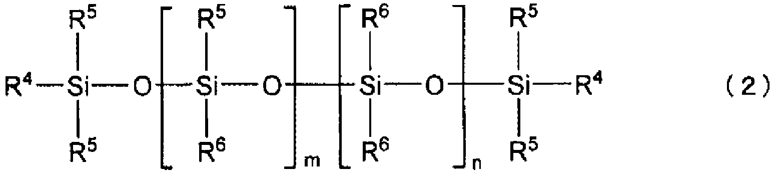

In Formula (2), R⁴ represents a substituted or unsubstituted alkyl group, alkenyl group, or aryl group having 1 to 10 carbon atoms, a hydrocarbon group including a combination thereof, or a hydride group. Examples of the alkyl group having 1 to 10 carbon atoms include a methyl group, an ethyl group, and a propyl group. In particular, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

In addition, R⁵ represents a substituted or unsubstituted alkyl group, alkenyl group, or aryl group having 1 to 10 carbon atoms, a hydrocarbon group including a combination thereof, or a hydride group. Examples of the alkyl group having 1 to 10 carbon atoms include a methyl group, an ethyl group, and a propyl group. In particular, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

In Formula (2), a plurality of R⁴'s may be independent from each other and may be the same as or different from each other. The same can be applied to R⁵. In this case, at least two or more among a plurality of R⁴' s and a plurality of R⁵' s represent a hydride group.

In addition, R⁶ represents a substituted or unsubstituted alkyl group or aryl group having 1 to 8 carbon atoms, or a hydrocarbon group including a combination thereof. Examples of the alkyl group having 1 to 8 carbon atoms include a methyl group, an ethyl group, and a propyl group. In particular, a methyl group is preferable. Examples of the aryl group having 1 to 8 carbon atoms include a phenyl group. A plurality of R⁶'s may be independent from each other and may be the same as or different from each other.

Examples of a substituent of R⁴, R⁵, and R⁶ in Formula (2) include a methyl group and a vinyl group. From the viewpoint of preventing a crosslinking reaction in the molecule, a methyl group is preferable.

Further, m and n each independently represent the number of repeating units forming the linear organohydrogen polysiloxane (B1) represented by Formula (2), m represents an integer of 2 to 150, and n represents an integer of 2 to 150. It is preferable that m represents an integer of 2 to 100 and n represents an integer of 2 to 100.

As the linear organohydrogen polysiloxane (B1), only one kind may be used alone, or two or more kinds may be used in combination.

The branched organohydrogen polysiloxane (B2) has a branched structure and thus is a component that largely contributes to the formation of a structure of the crosslinking density in the silicone rubber system by forming a region having a high crosslinking density. In addition, as in the linear organohydrogen polysiloxane (B1), the branched organohydrogen polysiloxane (B2) is a polymer that a structure (=Si-H) in which hydrogen is directly bonded to Si and that is obtained by a hydrosilylation reaction of the vinyl group in the vinyl group-containing organopolysiloxane (A) and a vinyl group in a component mixed in the silicone rubber-based curable composition to crosslink the components.

In addition, the specific gravity of the branched organohydrogen polysiloxane (B2) is in a range of 0.9 to 0.95.

Further, it is preferable that, typically, the branched organohydrogen polysiloxane (B2) does not have a vinyl group. As a result, the crosslinking reaction in the molecule of the branched organohydrogen polysiloxane (B2) can be reliably prevented from progressing.

In addition, it is preferable that the branched organohydrogen polysiloxane (B2) is represented by Average Compositional Formula (c) described below.

Average Compositional Formula (c) (Hₐ(R⁷)₃₋ₐSiO_{1/2})ₘ(SiO_{4/2})ₙ

(In Formula (c), R⁷ represents a monovalent organic group, a represents an integer of 1 to 3, m represents the number of Hₐ(R⁷)₃₋ₐSiO_{1/2} units, and n represents the number of SiO_{4/2} units.)

In Formula (c), R⁷ represents a monovalent organic group, and represents preferably a substituted or unsubstituted alkyl group or aryl group having 1 to 10 carbon atoms, or a hydrocarbon group including a combination thereof. Examples of the alkyl group having 1 to 10 carbon atoms include a methyl group, an ethyl group, and a propyl group. In particular, a methyl group is preferable. Examples of the aryl group having 1 to 10 carbon atoms include a phenyl group.

In Formula (c), a represents the number of hydride groups (hydrogen atoms directly bonded to Si) which is an integer of 1 to 3 and preferably 1.

In addition, in Formula (c), m represents the number of Hₐ(R⁷)₃₋ₐSiO_{1/2} units, and n represents the number of SiO_{4/2} units.

The branched organohydrogen polysiloxane (B2) has a branched structure. The linear organohydrogen polysiloxane (B1) and the branched organohydrogen polysiloxane (B2) are different from each other in that whether the structure is linear or branched. A ratio of the number (R/Si) of alkyl groups R bonded to Si to the number of Si that is 1 is 1.8 to 2.1 in the linear organohydrogen polysiloxane (B1) and is 0.8 to 1.7 in the branched organohydrogen polysiloxane (B2).

Since the branched organohydrogen polysiloxane (B2) has a branched structure, the amount of residues is 5% or higher, for example, during heating to 1000°C at a temperature increase rate of 10 °C/min in a nitrogen atmosphere. On the other hand, since the linear organohydrogen polysiloxane (B1) is linear, the amount of residues after heating under the above-described conditions is substantially zero.

In addition, specific examples of the branched organohydrogen polysiloxane (B2) include a branched organohydrogen polysiloxane (B2) having a structure represented by the following Formula (3).

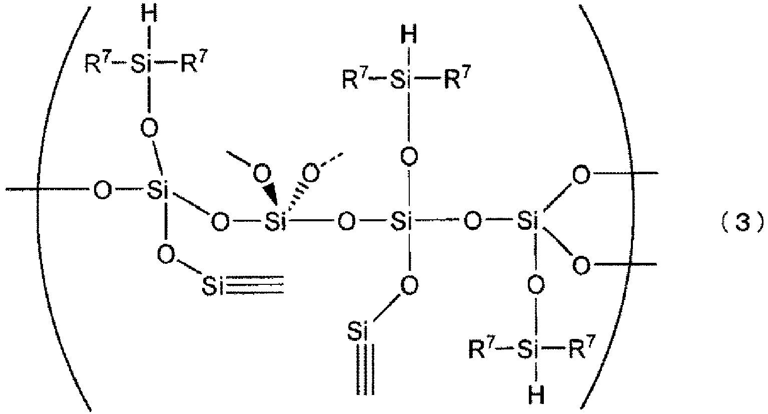

In Formula (3), R⁷ represents a substituted or unsubstituted alkyl group or aryl group having 1 to 8 carbon atoms, a hydrocarbon group including a combination thereof, or a hydrogen atom. Examples of the alkyl group having 1 to 8 carbon atoms include a methyl group, an ethyl group, and a propyl group. In particular, a methyl group is preferable. Examples of the aryl group having 1 to 8 carbon atoms include a phenyl group. Examples of a substituent of R⁷ include a methyl group.

In Formula (3), a plurality of R⁷'s may be independent from each other and may be the same as or different from each other.

In addition, in Formula (3), "-O-Si≡" represents that Si has a branched structure that spreads three-dimensionally.

As the branched organohydrogen polysiloxane (B2), only one kind may be used alone, or two or more kinds may be used in combination.

In addition, in each of the linear organohydrogen polysiloxane (B1) and the branched organohydrogen polysiloxane (B2), the amount of hydrogen atoms (hydride groups) directly bonded to Si is not particularly limited. In the silicone rubber-based curable composition, the total amount of hydride groups in the linear organohydrogen polysiloxane (B1) and the branched organohydrogen polysiloxane (B2) is preferably 0.5 to 5 mol and more preferably 1 to 3.5 mol with respect to 1 mol of vinyl group in the vinyl group-containing linear organopolysiloxane (A1). As a result, a crosslinked network can be reliably formed between the linear organohydrogen polysiloxane (B1) and the branched organohydrogen polysiloxane (B2), and the vinyl group-containing linear organopolysiloxane (A1).

### «Silica Particles (C)>>

The silicone rubber-based curable composition according to the embodiment optionally includes silica particles (C). As a result, the hardness or mechanical strength of the elastomer can be improved.

The silica particles (C) are not particularly limited. For example, fumed silica, pyrogenic silica, or precipitated silica is used. Among these, one kind may be used alone, or two or more kinds may be used in combination.

The specific surface area of the silica particles (C) measured using, for example, a BET method is, for example, preferably 50 to 400 m²/g and more preferably 100 to 400 m²/g. In addition, the average primary particle size of the silica particles (C) is, for example, preferably 1 to 100 nm and more preferably about 5 to 20 nm.

By using the silica particles (C) having the specific surface area and the average particle size in the above-described range, the hardness and the mechanical strength, in particular, the tensile strength of the formed silicone rubber can be improved.

### «Silane Coupling Agent (D)>>

The silicone rubber-based curable composition according to the embodiment may include a silane coupling agent (D).

The silane coupling agent (D) may include a hydrolyzable group. The hydrolyzable group is hydrolyzed into a hydroxyl group by water, and this hydroxyl group reacts with a hydroxyl group on the silica particles (C) in a dehydration synthesis reaction. As a result, the surfaces of the silica particles (C) can be modified.

In addition, the silane coupling agent (D) may include a silane coupling agent having a hydrophobic group. As a result, this hydrophobic group is added to the surfaces of the silica particles (C). Therefore, it is presumed, in the silicone rubber-based curable composition and the silicone rubber, the cohesion force between the silica particles (C) decreases (cohesion through a hydrogen bond formed by a silanol group decreases, and thus the dispersibility of the silica particles (C) in the silicone rubber-based curable composition is improved. As a result, the area of an interface between the silica particles (C) and a rubber matrix increases, and a reinforcing effect of the silica particles (C) increases. Further, it is presumed that, when the rubber matrix is deformed, the slipperiness of the silica particles (C) in the matrix is improved. By improving the dispersibility and slipperiness of the silica particles (C), the mechanical strength (for example, tensile strength or tear strength) of the silicone rubber by the silica particles (C) is improved.

Further, the silane coupling agent (D) may include a silane coupling agent having a vinyl group. As a result, a vinyl group is introduced into the surface of the silica particles (C). Therefore, when the silicone rubber-based curable composition is cured, that is, when a vinyl group in the vinyl group-containing organopolysiloxane (A) and a hydride group in the organohydrogen polysiloxane (B) react with each other in a hydrosilylation reaction such that a network (crosslinked structure) is formed, a vinyl group in the silica particles (C) gets involved with the hydrosilylation reaction with the hydride group in the organohydrogen polysiloxane (B). Therefore, the silica particles (C) are also incorporated into the network. As a result, low hardness and high modulus of the formed silicone rubber can be realized.

As the silane coupling agent (D), the silane coupling agent having a hydrophobic group and the silane coupling agent having a vinyl group can be used in combination.

Examples of the silane coupling agent (D) include a silane coupling agent represented by the following Formula (4).

Yₙ-Si-(X)₄₋ₙ... (4)

In Formula (4), n represents an integer of 1 to 3. Y represents any functional group of a hydrophobic group, a hydrophilic group, or a vinyl group, when n represents 1, Y represents a hydrophobic group, and when n represents 2 or 3, at least one of Y's represents a hydrophobic group. X represents a hydrolyzable group.

The hydrophobic group is an alkyl group or aryl group having 1 to 6 carbon atoms, or a hydrocarbon group including a combination thereof. Examples of the hydrophobic group include a methyl group, an ethyl group, a propyl group, and a phenyl group. In particular, a methyl group is preferable.

In addition, examples of the hydrophilic group include a hydroxyl group, a sulfonate group, a carboxyl group, and a carbonyl group. In particular, a hydroxyl group is preferable. The hydrophilic group may be included as a functional group . However, it is preferable that the hydrophilic group is not included from the viewpoint of imparting hydrophobicity to the silane coupling agent (D).

Further, examples of the hydrolyzable group include an alkoxy group such as a methoxy group or an ethoxy group, a chloro group, and a silazane group. In particular, a silazane group is preferable from the viewpoint of high reactivity with the silica particles (C). The silane coupling agent having a silazane group as the hydrolyzable group has a structure including two structures represented by (Yₙ-Si-) in Formula (4).

Specific examples of the silane coupling agent (D) represented by Formula (4) are as follows.

Examples of the silane coupling agent having a hydrophobic group as the functional group include: an alkoxysilane such as methyltrimethoxysilane, dimethyldimethoxysilane, phenyltrimethoxysilane, methyltriethoxysilane, dimethyldiethoxysilane, phenyltriethoxysilane, n-propyltrimethoxysilane, n-propyltriethoxysilane, hexyltrimethoxysilane, hexyltriethoxysilane, or decyltrimethoxysilane; a chlorosilane such as methyltrichlorosilane, dimethyldichlorosilane, trimethylchlorosilane, or phenyltrichlorosilane; and hexamethyldisilazane. In particular, a silane coupling agent having a trimethylsilyl group that includes one or more selected from the group consisting of hexamethyldisilazane, trimethylchlorosilane, trimethylmethoxysilane, and trimethylethoxysilane is preferable.

Examples of the silane coupling agent having a vinyl group as the functional group include: an alkoxysilane such as methacryloxypropyltriethoxysilane, methacryloxypropyltrimethoxysilane, methacryloxypropylmethyldiethoxysilane, methacryloxypropylmethyldimethoxysilane, vinyltriethoxysilane, vinyltrimethoxysilane, or vinylmethyldimethoxysilane; a chlorosilane such as vinyltrichlorosilane or vinylmethyldichlorosilane; and divinyltetramethyldisilazane. In particular, a silane coupling agent having a vinyl group-containing organosilyl group that includes one or more selected from the group consisting of methacryloxypropyltriethoxysilane, methacryloxypropyltrimethoxysilane, methacryloxypropylmethyldiethoxysilane, methacryloxypropylmethyldimethoxysilane, divinyltetramethyldisilazane, vinyltriethoxysilane, vinyltrimethoxysilane, and vinylmethyldimethoxysilane is preferable.

In addition, in a case where the silane coupling agent (D) includes two kinds including a silane coupling agent having a trimethylsilyl group and a silane coupling agent having a vinyl group-containing organosilyl group, it is preferable that hexamethyldisilazane is included as the silane coupling agent having a hydrophobic group and divinyltetramethyldisilazane is included as the silane coupling agent having a vinyl group.

In a case where the silane coupling agent (D1) having a trimethylsilyl group and the silane coupling agent (D2) having a vinyl group-containing organosilyl group are used in combination, a ratio between (D1) and (D2) is not particularly limited. For example, a weight ratio (D1) : (D2) is 1:0.001 to 1: 0.35, preferably 1:0.01 to 1:0.20, and more preferably 1:0.03 to 1:0.15. In the above-described numerical range, desired physical properties of the silicone rubber can be obtained. Specifically, a balance between the dispersibility of silica in the rubber and the crosslinkability of the rubber can be realized.

In the embodiment, the lower limit value of the content of the silane coupling agent (D) is preferably 1 mass% or higher, more preferably 3 mass% or higher, and still more preferably 5 mass% or higher with respect to 100 parts by weight of the total amount of the vinyl group-containing organopolysiloxane (A). In addition, the upper limit value of the content of the silane coupling agent (D) is preferably 100 mass% or lower, more preferably 80 mass% or lower, and still more preferably 40 mass% or lower with respect to 100 parts by weight of the total amount of the vinyl group-containing organopolysiloxane (A).

By adjusting the content of the silane coupling agent (D) to be lower limit value or higher, the adhesion between the pillar portion including the elastomer and the conductive resin layer can be improved. In addition, the improvement of the mechanical strength of the silicone rubber can be promoted. In addition, by adjusting the content of the silane coupling agent (D) to be the upper limit value or lower, the silicone rubber can be made to have appropriate mechanical properties.

### <<Platinum or Platinum Compound (E)>>

The silicone rubber-based curable composition according to the embodiment may include platinum or platinum compound (E).

The platinum or platinum compound (E) is a catalyst component that functions as a catalyst during curing. The addition amount of the platinum or platinum compound (E) is the amount of the catalyst.

As the platinum or platinum compound (E), a well-known compound can be used, and examples thereof include platinum black, silica or carbon black on which platinum is supported, chloroplatinic acid or an alcohol solution of chloroplatinic acid, a complex salt of chloroplatinic acid and olefin, and a complex salt of chloroplatinic acid and vinylsiloxane.

As the platinum or platinum compound (E), only one kind may be used alone, or two or more kinds may be used in combination.

In the embodiment, the content of the platinum or platinum compound (E) in the silicone rubber-based curable composition refers to the amount of the catalyst and can be appropriately set. Specifically, the content of platinum group metal by weight is 0.01 to 1000 ppm and preferably 0.1 to 500 ppm with respect to 100 parts by weight of the total amount of the vinyl group-containing organopolysiloxane (A), the silica particles (C), and the silane coupling agent (D).

By adjusting the content of the platinum or platinum compound (E) to be the lower limit value or higher, the silicone rubber-based curable composition can be cured at an appropriate rate. In addition, by adjusting the content of the platinum or platinum compound (E) to be the upper limit value or lower, a reduction in manufacturing costs can be promoted.

### «Water (F)>>

In addition, the silicone rubber-based curable composition according to the embodiment may include water (F) in addition to the components (A) to (E).

The water (F) is a component that functions as a dispersion medium for dispersing the respective components in the silicone rubber-based curable composition and contributes to the reaction between the silica particles (C) and the silane coupling agent (D) . Therefore, in the silicone rubber, the silica particles (C) and the silane coupling agent (D) can be more reliably linked to each other, and uniform properties can be exhibited as a whole.

### (Other Components)

Further, the silicone rubber-based curable composition according to the embodiment may further include other components in addition to the components (A) to (F) . Examples of the other components include an inorganic filler other than the silica particles (C) such as diatomaceous earth, iron oxide, zinc oxide, titanium oxide, barium oxide, magnesium oxide, cerium oxide, calcium carbonate, magnesium carbonate, zinc carbonate, glass wool, or mica, and an additive such as a reaction inhibitor, a dispersant, a pigment, a dye, an antistatic agent, an antioxidant, a flame retardant, or a thermal conductivity enhancing agent.

The conductive solution according to the embodiment includes the conductive filler and a solvent in addition to the silicone rubber-based curable composition not including the conductive filler.

As the solvent, various well-known solvents can be used. For example, a high boiling point solvent can be included. Among these, one kind may be used alone, or two or more kinds may be used in combination.

Examples of the solvent include: an aliphatic hydrocarbon such as pentane, hexane, cyclohexane, heptane, methylcyclohexane, ethylcyclohexane, octane, decane, dodecane, or tetradecane; an aromatic hydrocarbon such as benzene, toluene, ethylbenzene, xylene, trifluoromethylbenzene, or benzotrifluoride; an ether such as diethyl ether, diisopropyl ether, dibutyl ether, cyclopentyl methyl ether, cyclopentyl ethyl ether, ethylene glycol dimethyl ether, ethylene glycol diethyl ether, diethylene glycol dimethyl ether, 1,4-dioxane, 1,3-dioxane, or tetrahydrofuran; a haloalkane such as dichloromethane, chloroform, 1,1-dichloroethane, 1,2-dichloroethane, 1,1,1-trichloroethane, or 1,1,2-trichloroethane; a carboxylic acid amid such as N,N-dimethylformamide or N,N-dimethylacetamide; and a sulfoxide such as dimethyl sulfoxide or diethyl sulfoxide. Among these, one kind may be used alone, or two or more kinds may be used in combination.

By adjusting the solid content in the solution, the conductive solution can be made to have an appropriate viscosity for various coating methods such as spray coating or dip coating.

In addition, in a case where the conductive solution includes the conductive filler and the silica particles (C), the lower limit value of the content of the silica particles (C) in the conductive resin layer 30 is, for example, 1 mass% or higher, preferably 3 mass% or higher, and more preferably 5 mass% or higher with respect to 100 mass% of the total amount of the silica particles (C) and the conductive filler. As a result, the mechanical strength of the conductive resin layer 30 can be improved. On the other hand, the upper limit value of the content of the silica particles (C) in the conductive resin layer 30 is, for example, 20 mass% or lower, preferably 15 mass% or lower, and more preferably 10 mass% or lower with respect to 100 mass% of the total amount of the silica particles (C) and the conductive filler. As a result, a balance between the conductivity and the mechanical strength or flexibility in the conductive resin layer 30 can be realized.

An example of a method of manufacturing the biomedical electrode 100 according to the embodiment may include the following steps.

First, the silicone rubber-based curable composition is molded using a mold by hot press molding to obtain a molded body including the plate-shaped support portion 10 and the pillar portion 20 (molding step).

Next, the conductive wire 60 is inserted into the pillar portion 20 (conductive wire insertion step). For example, the conductive wire 60 can be made to pass the inside of the pillar portion 20 using a needle. Mass production can be performed using a sewing machine.

In the molding step, the silicone rubber-based curable composition 60 may be introduced into a molding space where the conductive wire is arranged such that insert molding of pressurizing and heating the composition is used.

Next, the distal end portion of the pillar portion 20 of the obtained molded body may be dipped in the conductive solution and may be heated and dried (distal end coating step).

Next, post-curing (annealing step) is performed at predetermined temperature under temperature conditions.

As a result, the biomedical electrode 100 can be manufactured.

After the molding step, before the conductive wire insertion step, the distal end portion 26 of the pillar portion 20 may be cut in a desired shape to form the inclined surface 28. The inclined surface 28 may be formed on the distal end portion 26 of the pillar portion 20 by molding instead of cutting.

The biomedical electrode 100 according to the embodiment can detect a bioelectric signal generated from organic activities of a brain, a heart, a muscle, a nerve, or the like. The biomedical electrode 100 has flexibility and excellent wearability on a scalp, and thus can be suitably used as an electroencephalographic electrode.

The application of an electroencephalographic electrode including the biomedical electrode 100 to a BMI (Brain Machine Interface) is expected.

In addition, the biomedical electrode 100 can be used as a dry sensor that is simple and can be repeatedly used instead of a wet sensor that requires application of gel to a measurement portion. In addition, the biomedical electrode 100 can have flexibility that can reduce pain or discomfort to a subject (user) as compared to a dry sensor of a metal pin type with a spring. In addition, the biomedical electrode 100 can be mounted on a wearable device due to a reduction in size.

A biomedical sensor according to the embodiment will be described.

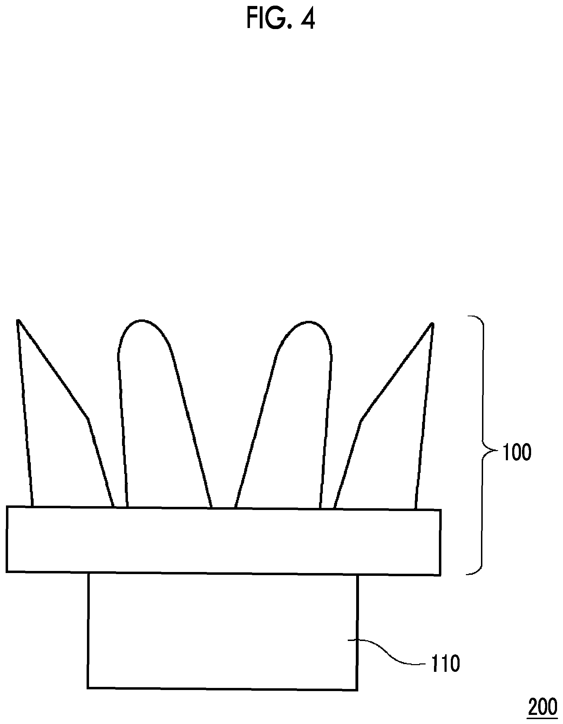

Fig. 4 is a schematic diagram showing the summary of a biomedical sensor 200.

The biomedical sensor 200 according to the embodiment includes the biomedical electrode 100 and may further include an external connection portion 110 connected to the biomedical electrode 100.

The external connection portion 110 may be detachably attached to the plate-shaped support portion 10 of the biomedical electrode 100 or may be fixed to the plate-shaped support portion 10.

From the viewpoint of durability, the external connection portion 110 includes at least an external electrode portion that is more rigid than the silicone rubber and has conductivity. The external electrode portion is formed of, for example, a metal. The external electrode portion can transmit a bioelectric signal detected by the biomedical electrode 100 to an external electronic component. The shape of the external electrode portion is not particularly limited and is configured to be a shape to which a connector or a wiring connectable to an electronic component can be attached. For example, the external connection portion 110 is configured with a metal snap button and has a structure that is electrically connected to an external wiring or an electrode of a substrate through a contact pin.

The biomedical sensor 200 may further include an electronic component that can be electrically connected through the external connection portion 110. As the electronic component, well-known components can be used depending on various uses. Examples of the electronic component include an amplifier, an AD converter, an impedance, a CPU, a memory, a communication circuit, and a battery. Among the examples, one or more components may be modularized on a circuit board. As a result, the biomedical sensor 200 can be used as a wearable device.

In addition, the biomedical sensor 200 may be used in combination with another sensor such as an acceleration sensor or a temperature sensor as the electronic component.

The biomedical sensor 200 includes one or more biomedical electrodes 100. The biomedical sensor 200 may be provided in an attachment jig to a living body such as a headgear or an arm band.

A biomedical signal measurement system according to the embodiment will be described.

The biomedical signal measurement system according to the embodiment includes the biomedical sensor 200. The biomedical signal measurement system may be a system (measurement device) that displays, analyzes, or stores data received from the biomedical sensor 200.

Hereinabove, the embodiment of the present invention has been described. However, the embodiment is an example of the present invention, and various configurations other than the above-described configuration can also be adopted.

Hereinafter, examples of the present invention will be described in detail. However, the present invention is not limited to the description of the examples.

Raw material components shown in Table 1 are as described below.

### (Vinyl Group-Containing Organopolysiloxane (A))

(A1-1): first vinyl group-containing linear organopolysiloxane (vinyl group content = 0.13 mol%, Mn = 227, 734, Mw = 573,903, IV value (dl/g) = 0.89), a vinyl group-containing dimethylpolysiloxane (the structure represented by Formula (1-1)) synthesized according to the following synthesis scheme 1
(A1-2): second vinyl group-containing linear organopolysiloxane: vinyl group content = 0.92 mol%, a vinyl group-containing dimethylpolysiloxane (the structure represented by Formula (1-1) where R¹ and R² represent a vinyl group) synthesized according to the following synthesis scheme 2

### (Organohydrogen Polysiloxane (B))

(B): organohydrogen polysiloxane: manufactured by Momentive Inc. "TC-25D"

### (Silica Particles (C))

(C): Silica fine particles (particle size: 7nm, specific surface area: 300 m²/g), manufactured by Nippon Aerosil Co., Ltd., "AEROSIL 300"

### (Silane Coupling Agent (D))

(D-1) : Hexamethyldisilazane (HMDZ), manufactured by Gelest Inc. "HEXAMETHYLDISILAZAE" (SIH6110.1)"
(D-2): divinyltetramethyldisilazane, manufactured by Gelest Inc., "1,3-DIVINYLTETRAMETHYLDISILAZANE (SID4612.0)"

### (Platinum or Platinum Compound (E))

(E) : platinum or a platinum compound: manufactured by Momentive Inc., "TC-25A"

### (Water (F))

(F) : pure water

### (Metal Powder (G))

(G1): silver powder, manufactured by Tokuriki Honten Co., Ltd, trade name "TC-101", median size d₅₀: 8.0 um, aspect ratio: 16.4, average long diameter: 4.6 µm

### (Synthesis of Vinyl Group-Containing Organopolysiloxane (A))

### [Synthesis scheme 1: Synthesis of First Vinyl

### Group-Containing Linear Organopolysiloxane (A1-1)]

The first vinyl group-containing linear organopolysiloxane (A1-1) was synthesized according to the following Formula (5).

That is, 74.7 g (252 mmol) of octamethylcyclotetrasiloxane and 0.1 g of potassium siliconate were added to 300 mL separable flask including a cooling tube and a mixing impeller where the atmosphere was replaced with Ar gas. The solution was heated and stirred at 120°C for 30 minutes. At this time, an increase in viscosity was observed.

Next, the solution was heated up to 155°C and was continuously stirred for 3 hours. After 3 hours, 0.1 g (0.6 mmol) of 1,3-divinyltetramethyldisiloxane was added and was further stirred at 155°c for 4 hours.

Further, after 4 hours, the solution was diluted with 250 mL of toluene, and was cleaned with water 3 times. After cleaning, the organic layer was cleaned with 1.5 L of methanol and was purified by reprecipitation to separate an oligomer and a polymer. The obtained polymer was dried at 60°C under reduced pressure overnight. As a result, the first vinyl group-containing linear organopolysiloxane (A1-1) was obtained (Mw = 573,903, Mn = 227,734). In addition, the vinyl group content calculated by H-NMR spectrum measurement was 0.13 mol%.

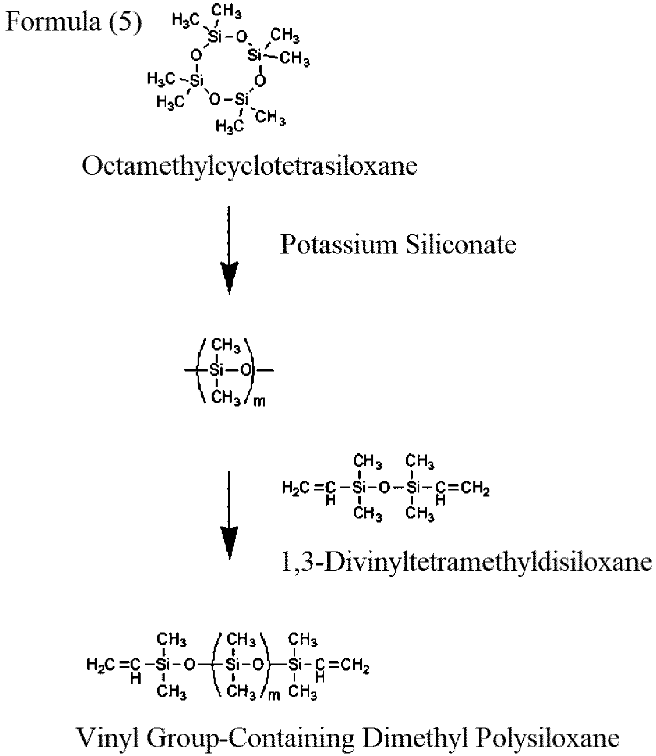

### [Synthesis scheme 2: Synthesis of Second Vinyl

### Group-Containing Linear Organopolysiloxane (A1-2)]

The second vinyl group-containing linear organopolysiloxane (A1-2)was synthesized as shown in the following Formula (6) using the same method as the synthesis step of (A1-1) , except that 74.7 g (252 mmol) of octamethylcyclotetrasiloxane and 0.86 g (2.5 mmol) of 2,4,6,8-tetraethenyl-2,4,6,8-tetravinylcyclotetrasiloxane were used in the synthesis step of (A1-1) . In addition, the vinyl group content calculated by H-NMR spectrum measurement was 0.92 mol%.

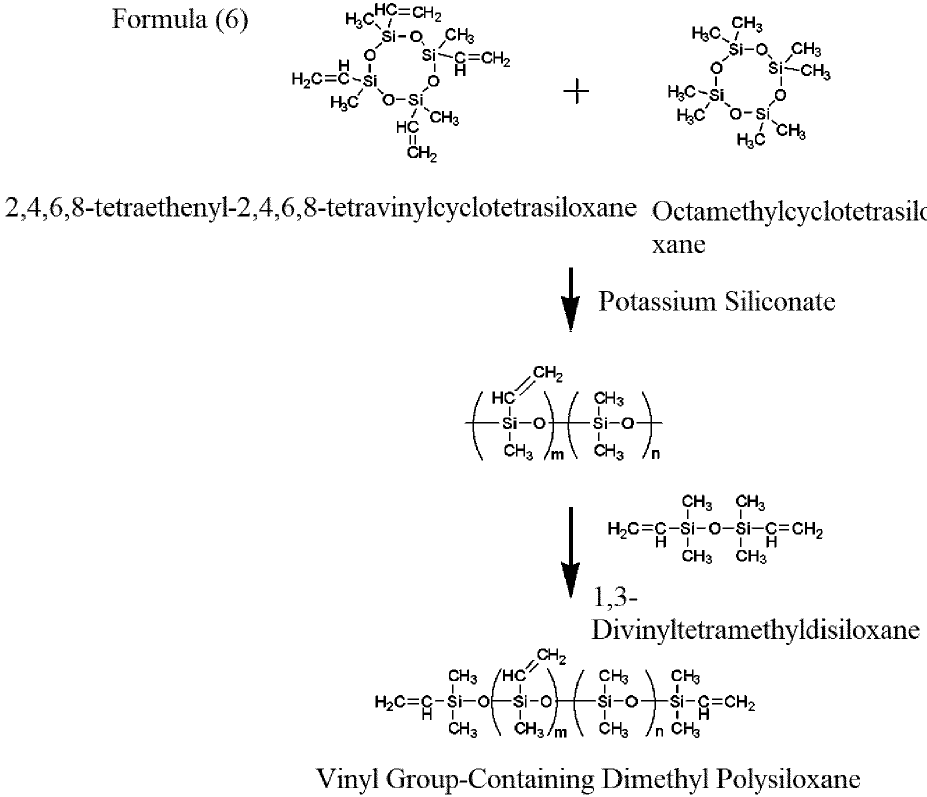

### <Preparation of Silicone Rubber-Based Curable Composition>

The silicone rubber-based curable composition was prepared as follows.

First, a mixture including 90% of the vinyl group-containing organopolysiloxane (A), the silane coupling agent (D), and the water (F) at a ratio shown in Table 1 below was kneaded in advance. Next, the silica particles (C) were further added to the mixture and kneaded. As a result, a kneaded material (silicone rubber compound) was obtained.

Here, the kneading after the addition of the silica particles (C) was performed through a first step of kneading the components under conditions of 60°C to 90°C in a nitrogen atmosphere for the coupling reaction and a second step of kneading the components under conditions of 160°C to 180°C for 2 hours in a reduced pressure atmosphere for removing the by-product (ammonia). Next, the kneaded material was cooled, the remaining 10% of the vinyl group-containing organopolysiloxane (A) was dividedly added twice, and the components were kneaded for 20 minutes.

Next, the organohydrogen polysiloxane (B) and the platinum or platinum compound (E) were added to 100 parts by weight of the obtained kneaded material (silicone rubber compound) at a ratio shown in Table 1 below, and the components were kneaded with a roll. As a result, the silicone rubber-based curable compositions A and B (elastomer compositions) were obtained.

**[Table 1]**

| | | | | Unit | A |
|---|---|---|---|---|---|
| Silicone Rubber-Based Curable Composition | Silicone Rubber Compound (Kneaded Material) | Vinyl Group-Containing Organopolysiloxane (A) | (A1-1) | Part by Weight | 80 |
| | | | (A1-2) | | 20 |
| | | Silica Particles (C) | | | 25 |
| | | Silane Coupling Agent (D) | (D-1) | | 10.0 |
| | | | (D-2) | | 0.5 |
| | | Water (F) | | | 5.25 |
| | Catalyst | Platinum or Platinum Compound (E) | | Part by Weight (with respect to 100 Parts by Weight of Kneaded Material) | 0.5 |
| | Crosslinking Agent | Organohydrogen Polysiloxane (B) | | | 1.81 |

### <Preparation of Conductive Solution for Dip Coating>

13.7 parts by weight of the obtained silicone rubber-based curable composition A was dipped in 31.8 parts by weight of decane (solvent), the solution was stirred using a rotating and revolving mixer, 54.5 parts by weight of metal powder (G1) was added, and the components were kneaded with three rolls. As a result, a conductive paste (conductive solution for dip coating) was obtained.

### <Preparation of Conductive Solution for Spray Coating>

13.7 parts by weight of the obtained silicone rubber-based curable composition A was dipped in 31.8 parts by weight of decane (solvent), the solution was stirred using a rotating and revolving mixer, 54.5 parts by weight of metal powder (G1) was added, and the components were kneaded with three rolls. As a result, a resin varnish was obtained. Next, decane was added to the resin varnish such that the amount of decane was 2.5 times that of the resin varnish, and was stirred using a rotating and revolving mixer to dilute the resin varnish. As a result, a conductive solution for spray coating was obtained.

### <Preparation of Biomedical Electrode>

### (Example 1)

The silicone rubber-based curable composition A was heated and cured at 180°C and 10 MPa for 10 minutes using a mold including a molding space for the plate-shaped support portion and six pillar portions. As a result, a molded body where the plate-shaped support portion and the six pillar portions were integrated was obtained (molding step).

Next, the distal end portions of all the pillar portions were obliquely cut to form an inclined surface in the distal end portion (cutting step).

Using a sewing needle, a conductive wire A (manufactured by Mitsufuji Corporation, AGposs, thickness: 100d/34f, tensile elongation at break: 29.3%) was made to pass the inside of each of the pillar portions of the obtained molded body (conductive wire insertion step).

Next, only the distal end portion of the pillar portion (when the overall length of the pillar portion was represented by L, a region of about 1/2L from the distal end) was dipped in <Conductive Solution for Dip Coating> and was heated and dried at 120°C for 30 minutes (distal end coating step).

Next, post-curing was performed at 140°C for 2 hours (annealing step).

As a result, a biomedical electrode A shown in Figs. 1A and 1B was obtained.

As shown in Fig. 3A, in the distal end structure of the biomedical electrode A, the distal end of the conductive wire 60 protruded from the distal end 22 of the pillar portion 20, and was covered with the conductive resin layer 30.

### (Example 2)

A biomedical electrode B was obtained using the same method as that of Example 1, except that a conductive wire B (manufactured by Mitsufuji Corporation, AGposs, thickness: 70d/24f, tensile elongation at break: 27.9%) was used instead of the conductive wire A.

### (Example 3)

A biomedical electrode C was obtained using the same method as that of Example 1, except that a conductive wire C (manufactured by Nippon Seisen Co., Ltd., metal fiber, stainless steel fiber NASLON, SUS304, thickness: 0.22 mm, tensile elongation at break: 1.6%) was used instead of the conductive wire A.

### (Comparative Example 1)

A molded body was obtained by performing the molding step and the cutting step based on <Preparation of Biomedical Electrode> using the same method as that of Example 1 without performing the conductive wire insertion step.

<Conductive Solution for Spray Coating> described above was applied to the entire surface of the obtained molded body by spray coating and was heated and dried at 120°C for 30 minutes. As a result, a conductive resin layer was formed on the surface of the molded body.

Next, only the distal end portion of the pillar portion was dipped in <Conductive Solution for Dip Coating> and was heated and dried at 120°C for 30 minutes. Next, post-curing was performed at 140°C for 2 hours. As a result, a biomedical electrode D was obtained.

The obtained biomedical electrodes A to D were evaluated for the following evaluation items. The evaluation results are shown in Table 2.

### (Wearing Stability)

By performing the molding step, the cutting step, and the conductive wire insertion step based on <Preparation of Biomedical Electrode> using the same method as that of Example 1, a steel ball (manufactured by Tsubaki Nakashima Co., Ltd., high carbon chromium bearing steel) having a diameter ϕ of 2 mm was attached to the distal end portion for connection to the conductive wire. As a result, a biomedical electrode E according to Comparative Example 2 was obtained.

While pressing the distal ends of the six pillar portions of the obtained biomedical electrode E according to Comparative Example 2 against the back of the head of a subject, a probe of a push-pull gauge (manufactured by Nidec-Shimpo Corporation, trade name: Digital Force Gauge FGJN-2) was pressed against another surface of the biomedical electrode E opposite to the distal end portion at a constant load of 15 N. At this time, the subject felt pain and endured the pain only for a short period of time in the evaluation.

On the other hand, when the biomedical electrodes A to C according to Examples 1 to 3 were used, the subject felt the sense of contact but did not feel uneasy or did not feel pain in the evaluation. Accordingly, it was found that, in the biomedical electrodes A to C according to Examples 1 to 3, the wearing stability was higher than that of the biomedical electrode E according to Comparative Example 2.

**[Table 2]**

| | | Unit | Example 1 | Example 2 | Example 3 | Comparative |
|---|---|---|---|---|---|---|
| | | | | | | Example 1 |
| Biomedical Electrode | | | A | B | C | D |
| Pressing Pressure | 15N | kΩ | 71 | 74 | 70 | 75 |
| | 10N | | 82 | 85 | 81 | 102 |
| | 5N | | 100 | 104 | 103 | 116 |
| | 3N | | 112 | 110 | 113 | 123 |
| | Δ(15N-10N) | | 11 | 11 | 11 | 27 |
| | Δ(15N-3N) | | 41 | 36 | 43 | 48 |

### (Measurement Stability)

### <Preparation of Electroencephalographic System>

As shown in Fig. 4, the external connection portion 110 (a metal snap button having a structure where an end portion of a cable is mountable) was mounted on the second surface 14 of the biomedical electrode 100 obtained in <Preparation of Biomedical Electrode> described above. A disposable electrode cord (manufactured by Miyuki Giken Co., Ltd., trade name: AP-C131-015) and a portable electroencephalograph (manufactured by Miyuki Giken Co., Ltd., trade name: Polymate Mini AP-108) were electrically connected to the external connection portion 110 in this order to prepare an electroencephalographic system. The portable electroencephalograph was connected to a laptop computer via Bluetooth (registered tradename), and the contact resistance with the head was acquired using a waveform display program (manufactured by Miyuki Giken Co., Ltd., trade name: Mobile Acquisition Monitor). For the ground and the reference, a left ear lobe was used.

Next, a headgear (a headgear that has a node arrangement according to the ten-twenty electrode system of the International Federation that was molded using a 3D printer) for electroencephalography was worn in the head of a subject.

Next, while bringing the distal end portion of the biomedical electrode 100 into contact with the back of the head of the subject such that the distal end portions 26 of the six pillar portions 20 of the obtained biomedical electrode 100 were pressed against the back of the head (0 z) of the subject, a probe of a push-pull gauge (manufactured by Nidec-Shimpo Corporation, trade name: Digital Force Gauge FGJN-2) was pressed against the external connection portion 110 of the biomedical electrode 100 at a load of 15 N first. Next, while gradually reducing the pressure, the contact resistance (kΩ) was continuously measured at 10 N, 5 N, and 3 N to evaluate a change in contact resistance relative to the load. The results are shown in Table 2.

It was found from Table 2 that, in the biomedical electrode according to Example 1, the amount of change (Δ(15 N - 10 N)) in contact resistance during the initial stage of load change was suppressed to be small and the amount of change (Δ(15 N - 3 N)) in contact resistance during a relatively wide load change was suppressed to be small as compared to Comparative Example 1.

Accordingly, it was found that, in the biomedical electrodes A to C according to Examples 1 to 3, the measurement stability was higher than that of the biomedical electrode D according to Comparative Example 1.

The present application claims priority based on Japanese Patent Application No. 2018-211725 filed on November 9, 2018.

## Claims

1. A biomedical electrode (100) comprising:
a plate-shaped support portion (10);
an elastic pillar portion (20) that is provided on a first surface (12) of the plate-shaped support portion (10);
a conductive resin layer (30) that is formed to cover a distal end (22) of the elastic pillar portion (20); and
a conductive wire (60) that is electrically connected to the conductive resin layer (30) and is arranged in the elastic pillar portion (20) from a distal end (22) side toward a base end side,
wherein the elastic pillar portion (20) is elastically deformable and is formed of an insulating elastic member including a silicone rubber, and
wherein a tensile elongation at break of the conductive wire (60) is 1% or higher and 50% or lower.

2. The biomedical electrode (100) according to claim 1,
wherein the conductive wire (60) is formed of conductive fiber.

3. The biomedical electrode (100) according to claim 2,
wherein the conductive wire (60) is formed of twisted yarn obtained by twisting a plurality of linear conductive fibers.

4. The biomedical electrode (100) according to claim 2 or 3,
wherein the conductive fiber includes one or more selected from the group consisting of metal fiber, metal-coated fiber, carbon fiber, conductive polymer fiber, conductive polymer-coated fiber, and conductive paste-coated fiber.

5. The biomedical electrode (100) according to any one of claims 1 to 4,
wherein when an overall length of the elastic pillar portion (20) is represented by L, the conductive resin layer (30) is formed in a region of 8/10L or less from the distal end (22) of the elastic pillar portion (20).

6. The biomedical electrode (100) according to any one of claims 1 to 5,
wherein the conductive resin layer (30) includes a conductive filler and a silicone rubber.

7. The biomedical electrode (100) according to claim 6,
wherein a content of the conductive filler is 30 mass% or higher and 90 mass% or lower with respect to 100 mass% of the silicone rubber.

8. The biomedical electrode (100) according to claim 6 or 7,
wherein the conductive filler includes one or more selected from the group consisting of metal particles, silver or silver chloride particles, metal fiber, metal-coated fiber, carbon black, acetylene black, graphite, carbon fiber, carbon nanotube, a conductive polymer, conductive polymer-coated fiber, and metal nanowire.

9. The biomedical electrode (100) according to any one of claims 1 to 8,
wherein a thickness of the conductive resin layer (30) is 5 µm or more and 200 µm or less.

10. The biomedical electrode (100) according to any one of claims 1 to 9,
wherein the biomedical electrode (100) is configured to be used as an electroencephalographic electrode.

11. A biomedical sensor (200) comprising the biomedical electrode (100) according to any one of claims 1 to 10.

12. A biomedical signal measurement system comprising the biomedical sensor (200) according to claim 11.

## Patentansprüche

1. Biomedizinische Elektrode (100), umfassend:
einen plattenförmigen Stützabschnitt (10);
einen elastischen Säulenabschnitt (20), der auf einer ersten Oberfläche (12) des plattenförmigen Stützabschnitts (10) vorgesehen ist;
eine leitfähige Harzschicht (30), die so ausgebildet ist, dass sie ein distales Ende (22) des elastischen Säulenabschnitts (20) bedeckt; und
einen leitfähigen Draht (60), der elektrisch mit der leitfähigen Harzschicht (30) verbunden ist und in dem elastischen Säulenabschnitt (20) von einer Seite des distalen Endes (22) zu einer Seite des Basisendes hin angeordnet ist,
wobei der elastische Säulenabschnitt (20) elastisch verformbar ist und aus einem isolierenden elastischen Element gebildet ist, das einen Silikonkautschuk enthält, und
wobei die Zugdehnung beim Bruch des leitenden Drahtes (60) 1% oder mehr und 50% oder weniger beträgt.

2. Biomedizinische Elektrode (100) nach Anspruch 1,
wobei der leitfähige Draht (60) aus leitfähiger Faser gebildet ist.

3. Biomedizinische Elektrode (100) nach Anspruch 2,
wobei der leitfähige Draht (60) aus verdrilltem Garn gebildet ist, das durch Verdrillen einer Vielzahl von linearen leitfähigen Fasern erhalten wird.

4. Biomedizinische Elektrode (100) nach Anspruch 2 oder 3,
wobei die leitfähige Faser eine oder mehrere, ausgewählt aus der Gruppe bestehend aus Metallfaser, metallbeschichteter Faser, Kohlenstofffaser, leitfähiger Polymerfaser, leitfähiger polymerbeschichteter Faser und leitfähiger pastenbeschichteter Faser, umfasst.

5. Biomedizinische Elektrode (100) nach einem der Ansprüche 1 bis 4,
wobei, wenn eine Gesamtlänge des elastischen Säulenabschnitts (20) durch L dargestellt wird, die leitfähige Harzschicht (30) in einem Bereich von 8/10L oder weniger vom distalen Ende (22) des elastischen Säulenabschnitts (20) gebildet ist.

6. Biomedizinische Elektrode (100) nach einem der Ansprüche 1 bis 5,
wobei die leitfähige Harzschicht (30) einen leitfähigen Füllstoff und einen Silikonkautschuk enthält,

7. Biomedizinische Elektrode (100) nach Anspruch 6,
wobei der Gehalt des leitfähigen Füllstoffs 30 Masse-% oder mehr und 90 Masse-% oder weniger, bezogen auf 100 Masse-% des Silikonkautschuks, beträgt.

8. Biomedizinische Elektrode (100) nach Anspruch 6 oder 7,
wobei der leitfähige Füllstoff eines oder mehrere, ausgewählt aus der Gruppe bestehend aus Metallpartikeln, Silber- oder Silberchloridpartikeln, Metallfasern, metallbeschichteten Fasern, Ruß, Acetylenschwarz, Graphit, Kohlenstofffasern, Kohlenstoffnanoröhren, einem leitfähigen Polymer, leitfähigen polymerbeschichteten Fasern und Metallnanodrähten, umfasst.

9. Biomedizinische Elektrode (100) nach einem der Ansprüche 1 bis 8,
wobei die Dicke der leitfähigen Harzschicht (30) 5 µm oder mehr und 200 µm oder weniger beträgt.

10. Biomedizinische Elektrode (100) nach einem der Ansprüche 1 bis 9,
wobei die biomedizinische Elektrode (100) zur Verwendung als elektroenzephalographische Elektrode konfiguriert ist.

11. Biomedizinischer Sensor (200), umfassend die biomedizinische Elektrode (100) nach einem der Ansprüche 1 bis 10.

12. Biomedizinisches Signalmesssystem, umfassend den biomedizinischen Sensor (200) nach Anspruch 11.

## Revendications

1. Électrode biomédicale (100) comportant :
une partie de support en forme de plaque (10) ;
une partie de colonne élastique (20) qui est disposée sur une première surface (12) de la partie de support en forme de plaque (10) ;
une couche de résine conductrice (30) qui est formée de manière à recouvrir une extrémité distale (22) de la partie de colonne élastique (20) ; et
un fil conducteur (60) qui est électriquement connecté à la couche de résine conductrice (30) et est agencé dans la partie de colonne élastique (20) depuis un côté de l'extrémité distale (22) vers un côté d'extrémité de base,
dans laquelle la partie de colonne élastique (20) est élastiquement déformable et est formée d'un élément élastique isolant incluant un caoutchouc silicone, et
dans laquelle un allongement à la rupture en traction du fil conducteur (60) est de 1 % ou plus et de 50 % ou moins.

2. Électrode biomédicale (100) selon la revendication 1,
dans laquelle le fil conducteur (60) est formé d'une fibre conductrice.

3. Électrode biomédicale (100) selon la revendication 2,
dans laquelle le fil conducteur (60) est formé d'un fil torsadé obtenu en torsadant une pluralité de fibres conductrices linéaires.

4. Électrode biomédicale (100) selon la revendication 2 ou 3,
dans laquelle la fibre conductrice inclut une ou plusieurs fibres choisies parmi le groupe constitué de fibre métallique, de fibre revêtue de métal, de fibre de carbone, de fibre de polymère conducteur, de fibre enrobée d'un polymère conducteur et de fibre enrobée d'une pâte conductrice.

5. Électrode biomédicale (100) selon l'une quelconque des revendications 1 à 4,
dans laquelle lorsqu'une longueur totale de la partie de colonne élastique (20) est représentée par L, la couche de résine conductrice (30) est formée dans une zone de 8/10L ou moins par rapport à l'extrémité distale (22) de la partie de colonne élastique (20).

6. Électrode biomédicale (100) selon l'une quelconque des revendications 1 à 5,
dans laquelle la couche de résine conductrice (30) inclut une charge conductrice et un caoutchouc silicone,

7. Électrode biomédicale (100) selon la revendication 6,
dans laquelle une teneur en charge conductrice est de 30 % en masse ou plus et de 90 % en masse ou moins par rapport à 100 % en masse du caoutchouc silicone,

8. Électrode biomédicale (100) selon la revendication 6 ou 7,
dans laquelle la charge conductrice inclut un ou plusieurs éléments choisis parmi le groupe constitué de particules métalliques, de particules d'argent ou de chlorure d'argent, de fibre métallique, de fibre revêtue de métal, de noir de carbone, de noir d'acétylène, de graphite, de fibre de carbone, de nanotube de carbone, de polymère conducteur, de fibre enrobée d'un polymère conducteur et de nanofil métallique,

9. Électrode biomédicale (100) selon l'une quelconque des revendications 1 à 8,
dans laquelle une épaisseur de la couche de résine conductrice (30) est de 5 µm ou plus et de 200 µm ou moins.

10. Électrode biomédicale (100) selon l'une quelconque des revendications 1 à 9,
dans laquelle l'électrode biomédicale (100) est configurée pour être utilisée comme une électrode électro-encéphalographique.

11. Capteur biomédical (200) comportant l'électrode biomédicale (100) selon l'une quelconque des revendications 1 à 10.

12. Système de mesure de signal biomédical comportant le capteur biomédical (200) selon la revendication 11.
